# EUROPEAN PATENT SPECIFICATION

(11) **EP 3 705 475 B1**
(45) Date of publication and mention of the grant of the patent: **30.08.2023**
(21) Application number: 19382163.4
(22) Date of filing: 05.03.2019
(51) Int. Cl.: C07D 209/60, A61K 31/405, A61K 9/20

(54) **HIGH-DENSITY L-TRYPTOPHAN, ITS PREPARATION AND USES THEREOF**
HOCHDICHTES L-TRYPTOPHAN, DESSEN HERSTELLUNG UND VERWENDUNGEN DAVON
L-TRYPTOPHANE HAUTE DENSITÉ, SA PRÉPARATION ET SES UTILISATIONS

(43) Date of publication of application: 09.09.2020
(73) Proprietor: Tradichem Industrial Services, S.L., 28108 Alcobendas (ES)
(72) Inventor: MARAÑÓN MAROTO, José Ángel, 28240 HOYO DE MANZANARES (ES); MORENO PUENTE, Patricia, 28007 MADRID (ES); LOZANO MARTÍN, Cristina, 28251 RIVAS VACIAMADRID (ES); MANCHA AVELLÁN, María, 28006 MADRID (ES)
(74) Representative: Sugrañes, S.L.P.

(56) References cited:
- EP-A1- 2 179 731
- US-A- 4 675 339
- US-A1- 2004 213 838
- AJINOMOTO ANIMAL NUTRITION NORTH AMERCA, INC.: "TECHNICAL DATA SHEET for L-Tryptophan [Feed Grade, 98%]", , 27 February 2019 (2019-02-27), XP002792755, Retrieved from the Internet: URL:http://www.lysine.com/pdf/Ajinomoto_He artland_L-Tryptophan_TDS.pdf [retrieved on 2019-07-05]
- TENG Y ET AL: "Systematical approach of formulation and process development using roller compaction", EUROPEAN JOURNAL OF PHARMACEUTICS AND BIOPHARMACEUTICS, ELSEVIER SCIENCE PUBLISHERS B.V., AMSTERDAM, NL, vol. 73, no. 2, 1 October 2009 (2009-10-01), pages 219-229, XP026652682, ISSN: 0939-6411, DOI: 10.1016/J.EJPB.2009.04.008 [retrieved on 2009-05-03]
- P. S. GAWARKAR ET AL.: "Roller compaction: an overview", INT. J. UNIVERSAL PHARM. BIO SCIENCES, vol. 4, no. 3, 1 May 2015 (2015-05-01), pages 67-76, XP002792756,

## Description

### Technical field

The present invention relates to high-density L-tryptophan which is particularly suitable for the manufacture of pharmaceutical dosage forms.

### Technical background

L-tryptophan is an essential aminoacid which has to be supplied through the diet, by ingested proteins. L-tryptophan is the least abundant amino acid in most proteins, accounting, on the average, for 1 to 1.5% of the total amino acids in typical plant (1%) and animal (1.5%) proteins.

Ingested tryptophan is used not only for the biosynthesis of proteins, but it is also the precursor of serotonin, melatonin and niacin (or vitamin B3).

Due to the relatively low content of tryptophan in the diet and the diversity of physiological functions in which it is involved, the ingestion of supplementary tryptophan is recommended in some cases as it brings some remarkable therapeutic benefits.

Thus, tryptophan has been used as a therapeutic agent for more than 30 years for treating conditions such as pain, insomnia, premenstrual syndrome, schizophrenia, affective disorders and behavioural disorders, as disclosed, for example, in Tryptophan. Biochemical and Health Implications, H. Sidransky, CRC Series in Modern Nutrition, CRC Press, 2002, ISBN 0-8493-8568-7, Chapter 8: Pharmacology and selected therapeutic uses, 189-207. The mean rationale for its therapeutic use is because the ingestion of tryptophan increases brain tryptophan concentrations, which stimulates the synthesis and release of the neurotransmitter serotonin, from which it is derived. Such increase in serotonin function is related to improvement of mood and sleep, for example.

Therefore, tryptophan supplements are widely available in the market, mainly in the form of tablets or capsules. The recommended doses depend on the indication, but generally range from about 500 mg to several grams per day.

The relatively high doses of tryptophan required in therapeutics make it necessary the formulation of high-dosage pharmaceutical forms. Thus, most available dosage forms contain doses of at least 300 mg, or preferably higher, for example, 400 mg, 500 mg or even 1000 mg per dose.

Furthermore, it has been disclosed that certain dosage forms provide increased bioavailability of L-tryptophan. Thus, for example, in the patent application DE2824362-A1 it is disclosed that formulations in the form of soft gelatin capsules, wherein L-tryptophan is mixed with vegetable oils, provide better bioavailability and longer duration of action than comparative formulations containing the same amount of L-tryptophan, but in the form of tablets or hard gelatin capsules. However, the formulation of L-tryptophan in the form of soft gelatin capsules requires the use of relatively high amounts of excipients and, consequently, relatively large dosage forms are obtained.

The US patent 4,675,339 discloses a process for the crystallization of L-amino acids, including L-tryptophan, to obtain granules of smaller specific volume and improved fluidity compared to conventional amino acid crystals, said process comprising adding a small amount of a polymer to an aqueous solution of the amino acid and subsequently precipitating the crystals.

Therefore, there is the need of further alternatives to provide suitable pharmaceutical formulations comprising L-tryptophan. In particular, a reduction in the amount of excipient required for the formulation and the also the decrease of the volume of final dosage form would be desirable.

### Object of the invention

The object of the present invention is a process for obtaining compacted L-tryptophan.

Another aspect of the invention is compacted L-tryptophan obtainable with such process.

Another aspect of the invention is the use of compacted L-tryptophan for the preparation of pharmaceutical compositions.

Another aspect of the invention is a pharmaceutical composition comprising compacted L-tryptophan.

Another aspect of the invention a pharmaceutical composition comprising compacted L-tryptophan for use in therapy.

### Description of the drawings

Figure I represents the results of several compaction assays of L-tryptophan, as disclosed in Example 1 (assay A), performed with a roller compactor apparatus, using a roll gap of 0.5 mm and roll force of 14000-15000 Kgf. The x-axis represents the feed speed (in r.p.m.) and the y-axis represents the bulk density values (in g/ml).
Figure II represents the results of several compaction assays of L-tryptophan, as disclosed in Example 1 (assay B), performed with a roller compactor apparatus, using a roll gap of 0.5 mm and roll force of 6000-7000 Kgf. The x-axis represents the feed speed (in r.p.m.) and the y-axis represents the bulk density values (in g/ml).
Figure III represents the results of several compaction assays of L-tryptophan, as disclosed in Example 1 (assay C), performed with a roller compactor apparatus, using a roll gap of 0.5 mm and a feed speed of 1.15 rad/s (11 r.p.m.). The x-axis represents the roll force (in Kgf) and the y-axis represents the bulk density values (in g/ml).
Figure IV represents the results of several compaction assays of L-tryptophan, as disclosed in Example 1 (assay D), performed with a roller compactor apparatus, using a roll gap of 0.5 mm and a feed speed of 6.28 rad/s (60 r.p.m.). The x-axis represents the roll force (in Kgf) and the y-axis represents the bulk density values (in g/ml).
Figure V represents the results of several compaction assays of L-tryptophan, as disclosed in Example 1 (assay E), performed with a roller compactor apparatus, using a feed speed of 6.28 rad/s (60 r.p.m.) and a roll force of 137209.31-147009.97 N (14000-15000 Kgf). The x-axis represents the roll gap (in mm) and the y-axis represents the bulk density values (in g/ml).
Figure VI shows two soft-capsules prepared as disclosed in Reference Example and in Example 2, using low-density L-tryptophan and high-density L-tryptophan, respectively.

### Detailed description of the invention

The object of the present invention is a process for preparing substantially pure compacted L-tryptophan comprising roller compaction of L-tryptophan, wherein substantially pure means a purity of at least 98% and preferably of at least 98.5%, and wherein the L-tryptophan is compacted alone without adding any auxiliary excipient.

The authors of the present invention have surprisingly found that, firstly, L-tryptophan can be successfully compacted using a roller compaction method and, secondly, that compacted L-tryptophan thus obtained can be successfully used to prepare pharmaceutical forms which are less voluminous, and which require the use of less excipients for a given dose of L-tryptophan.

Along the present description, as well as in the claims, the terms "a," "an," or "the" not only include aspects with one member (singular), but also includes aspects with more than one member (plural).

The terms "about" or "approximately" referred to amounts, as used herein, are meant to include a certain deviation of the qualified amount, namely of ±5%.

The numerical ranges disclosed herein are meant to include any number falling within the ranges and also the lower and upper limits.

### L-Tryptophan

L-Tryptophan, also known simply as tryptophan, or by its IUPAC name (*2S*)-2-amino-3-(*1H*-indol-3-yl)propanoic acid (CAS number 73-22-3) is a nonpolar alpha-aminoacid containing an indole ring in the side chain.

L-tryptophan is commercially available from several sources. For the production of L-tryptophan, many kinds of chemical, enzymatic, and fermentation methods are known in the art and L-tryptophan obtained by any of them is suitable to be used in the present invention.

At present, commercial production of L-tryptophan mostly is performed through two different microbial processes: production from cheap carbon and nitrogen sources by fermentation and conversion from chemically synthesized precursors by enzymatic reactions, as is well known in the art, as disclosed, for example, in Ikeda M, L-Tryptophan production, in: Handbook of Corynebacterium glutamicum, Editors L. Eggling & M. Bott, Taylor & Francis, CRC Press, 2005, Chapter 21, 489-509. For the direct fermentation process, typically, a number of mutant bacterial strains derived from species such as *Escherichia coli, Bacillus subtilis,* and *Corynebacterium glutamicum* and its subspecies can be used. Any suitable fermentation medium may be used, as long it contains carbon sources and nitrogen sources. As carbon sources, different kinds of carbohydrates may be used, such as glucose, glycerol, fructose, sucrose, maltose, mannose, starch, starch hydrolyzate, molasses or polyalcohols. Typically, the processes include subsequent steps of purification and/or crystallization.

The bulk density, as used in the present description, is defined as the ratio of the mass to the volume of an untapped powder sample, as "poured". The bulk density is given in grams per millilitre (g/ml) and is typically calculated by "pouring" a known mass of powder into a graduated cylinder. Bulk density can be measured according to the method disclosed in the European Pharmacopoeia section 2.9.34 (*Bulk density and tapped density of powders),* for example, using the PT-SV100 Scott Volumeter (Pharma Test Apparatebau AG, Germany).

The conventional, commercially available L-tryptophan, as disclosed so far in the art, and obtained by the methods disclosed above, has a bulk density typically of less than 0.20 g/ml, usually comprised between 0.12 and 0.19 g/ml. Such L-tryptophan is designed herein as low-density tryptophan.

Low-density L-tryptophan has a number of drawbacks to be used for the preparation of pharmaceutical forms, namely, it has poor flowability and requires relatively high amount of excipients to prepare pharmaceutical dosage forms.

Low-density L-tryptophan as disclosed herein is used as starting material for preparing compacted, or high-density L-tryptophan.

Low-density L-tryptophan is available in the form of powder or granules, with particle size typically of less than 1.5 mm, or less than 1 mm. Typically, particles of desired size can be obtained by sieving, i.e., by passing through a sieve of desired mesh. Low-density L-tryptophan of any particle size is suitable to be used in the present invention.

Low-density L-tryptophan used as raw material for the present invention has typically a purity of at least 95%, preferably of at least 96%, more preferably of at least 97%, still more preferably of at least 98%, and still more preferably of at least 98.5%. Accordingly, the expression "substantially pure" as used herein referred to L-tryptophan, either to low-density L-tryptophan or to high-density L-tryptophan, means a degree of purity of at least 98%, and still more preferably of at least 98.5%.

The purity degree of L-tryptophan can be determined using methods known in the art, for example, by HPLC.

### Compaction process

Commercially available low-density tryptophan, as disclosed above, is used as starting material to prepare high-density L-tryptophan according to the present invention.

The compaction or densification process is performed using the roller compaction technology, which is well-known in the pharmaceutical technology field, namely, for the dry granulation of pharmaceutical powder mixtures. Such technology is widely described in different reference manuals in the art, for example, in R.W. Miller, Roller Compaction Technology, in: Handbook of Pharmaceutical Granulation Technology, Editor D.M. Parikh, Third Edition, Informa Healthcare USA, 2010, Chapter 8, 163-182. Briefly, in the roller compaction process, the powder being compacted is squeezed between two counter-rotating rolls to form a compressed sheet, which is subsequently milled into granules.

There are several parameters that can be adjusted in order to optimize the compaction process, namely, feed speed, roll speed, roll pressure, roll gap and mill speed.

As is well-known in the art, the feeding system in the roller compactor has the task of feeding the powder to be compacted to the rolls and must ensure a uniform flow of material in order to continuously fill the nip between the rolls as they rotate. It can be a gravity feeder, wherein the powder is fed vertically by gravity, or a force feeder, wherein the powder is pushed towards the rolls by one or several screws. Preferably, screw feeding is used in the present invention, and the feeder can be vertical, horizontal or inclined. In one preferred embodiment, the screw feeder is vertical and is situated in the upper part of the equipment, over the rolls. The screw feeder may be a hopper or analogous container containing an endless screw.

The feed speed can affect the properties of the compacted product, and thus it can be controlled by adjusting the feed screw r.p.m. The feed speed is preferably constant, and is typically comprised between about 1.04 and about 7.33 rad/s (between about 10 and about 70 r.p.m.). In a preferred embodiment of the invention, the feed speed is comprised between 3.14 and 7.33 rad/s (between about 30 and 70 r.p.m.). In one embodiment, the feed speed is selected from about 3.14 rad/s (30 r.p.m.), about 4.18 rad/s (40 r.p.m.), about 5.23 rad/s (50 r.p.m.), about 6.28 rad/s (60 r.p.m.) and about 7.33 rad/s (70 r.p.m).

Preferably, low-density L-tryptophan raw material is sent for compaction into the screw feeder from another hopper or reservoir containing the product, for example, using vacuum for sucking the powder and sending it to the screw feeder in a controlled way. The sucking of the raw material and its delivery into the screw feeder can be controlled in an automatic way, for example, by placing a probe inside the feeder that activates the feeding of the raw material when necessary.

The roller unit consists of two equal diameter counter rotating rolls through which the powder is passed to get compacted. The two rolls can be mounted in horizontal, vertical or inclined position.

The roll speed can also be adjusted and may affect the characteristics of the compacted product. The roll speed typically is comprised between about 0.52 and about 3.66 rad/s (between about 5 and about 35 r.p.m.). In a preferred embodiment of the invention the roll speed is comprised between 0.52 and 2.09 rad/s (5 and 20 r.p.m.), more preferably comprised between 0.52 and 1.57 rad/s (between 5 and 15 r.p.m.), and still more preferably is about 0.73 rad/s (7 r.p.m.).

Another adjustable parameter is the roll gap, i.e., the distance between the two rolls. It typically ranges from about 0.1 mm to about 1.5 mm, preferably from 0.3 mm to 0.9 mm, more preferably from 0.4 to 0.7 mm, and still more preferably is about 0.5 mm.

Generally, one roll is fixed, while the other is movable and exerts pressure on the fixed roll by means of a hydraulic pressure control system. The horizontal pressure/force between rolls can be also adjusted and may also have influence on the properties of the end-product. The roll pressure/force typically ranges from about 39202.66 to about 176411.97 N (form about 4000 to about 18000 KgD, preferably from about 49003.32 to about 156810.64 N (from about 5000 to about 16000 Kgf). In an embodiment of the invention, the pressure/force between rolls ranges from 117607.98 to 166611.30 N (from 12000 to 17000 KgD, preferably from 127408.64 to 156810.64 N (from 13000 to 16000 KgD, more preferably from 137209.31 to 147009.97 N (from 14000 to 15000 Kgf).

The roll surface may be either smooth or non-smooth, i.e., may have rough pattern surface, for example, fluted, grooved or knurled surface. The roll surface of the two rolls may be identical or different.

In one embodiment of the invention, both rolls have smooth surface. In another embodiment of the invention, one roll has a smooth surface and the other has a non-smooth surface, for example, fluted, grooved or knurled surface.

Once low-density L-tryptophan has been compacted by passing through the two rolls, it is transformed in a compressed sheet or ribbon, which is subsequently sent to the granulation module. Such granulation module comprises one or more mills which break the compacted ribbon into compacted granules which are sieved through the desired mesh size to obtain granules of the desired particle size. In this step of the compaction process, the speed of the granulation mill can also be adjusted, which is typically comprised in the range of from about 5.23 to about 52.35 rad/s (from about 50 to about 500 r.p.m.), preferably from 6.28 to 20.94 rad/s (60 to 200 r.p.m.).

Typically, the roller compaction technology is used in the pharmaceutical field as a step in the preparation of solid pharmaceutical dosage forms, as an alternative to the wet granulation technology for preparing granules containing the the active ingredient together with excipients. Therefore, typical roller compaction formulations include the active ingredient together with pharmaceutical excipients usually selected from fillers, diluents, binders, disintegrants, lubricants, glidants, surfactants and mixtures thereof, as disclosed in R.W. Miller *op. cit.* Conversely, the compaction of active ingredients alone, without the aid of adequate excipients, is regarded as problematic, and is generally considered unsuitable in order to prepare final pharmaceutical formulations. Consequently, this approach is rarely disclosed in the art. Furthermore, the compaction of powders using the roller compaction methodology is generally acknowledged to involve a mixture of bonding mechanisms, through consecutive processes of rearrangement, deformation, fragmentation and bonding of the particles. Such process is highly dependent on the nature of the powder particles being compressed and is therefore highly unpredictable.

The authors of the present invention have surprisingly found that particles of L-tryptophan can be successfully compacted on their own, using the roller compaction methodology, without the need of adding auxiliary excipients. Using this method, L-tryptophan can be compacted to obtain increases in the bulk density generally higher than 20% relative to the bulk density of L-tryptophan before compaction; preferably the increase in bulk density is higher than 25%, more preferably higher than 35%, still more preferably higher than 45%, still more preferably higher than 55%, still more preferably higher than 65%, still more preferably higher than 75%, still more preferably higher than 85%, and still more preferably higher than 100%; it is possible to obtain increases in the bulk density of even more than 110%, 120%, 130% or 140%, for example. L-tryptophan after the compaction process is designed herein as high-density L-tryptophan or compacted L-tryptophan interchangeably.

Remarkably, after the densification process, L-tryptophan maintains its chemical integrity, so there is neither loss in purity nor increase in impurity occurrence. Therefore, the densification process according to the present invention allows the preparation of substantially pure compacted L-tryptophan, which has substantially the same purity degree as the starting material.

Another aspect of the invention is compacted L-tryptophan which is obtainable by a roller compaction process, as disclosed above.

The compacted tryptophan according to the present invention has a bulk density equal to or higher than 0.32 g/ml, still more preferably higher 0.34 g/ml, still more preferably 0.36 g/ml, still more preferably higher than 0.38 g/ml, still more preferably higher than 0.40 mg/ml, still more preferably higher than 0.42 g/ml, still more preferably higher than 0.44 g/ml, and still more preferably higher than 0.46 g/ml. Typically, the highest bulk density of L-tryptophan achieved with the present method is about 0.65 g/ml.

The size of the particles of obtained high-density L-tryptophan can be adjusted using a sieve of suitable mesh size to obtain particles of the desired size, namely, under a specific size determined by the sieve opening. Typically, the mesh size of the sieve is adjusted to obtain particles of less than 1.5 mm, preferably less than 1.4 mm, more preferably less than 1.3 mm, still more preferably less than 1.2 mm, still more preferably less than 1.1 mm and still more preferably less than 1 mm. Other mesh sizes are also suitable, for example, less than 900 microns, less than 800 microns, less than 700 microns, less than 600 microns, or less than 500 microns, among others.

The selected size of the high-density L-tryptophan particles does not have any substantial influence in the bulk density of the product. It was observed that the increase in the bulk density of L-tryptophan was achieved with the process of the invention while maintaining substantially the same particle size as in the low-density L-tryptophan employed as starting material.

The compacting process can optionally be repeated more than one time, i.e., the once compacted L-tryptophan can be sent again for compaction in order to increase its density. However, it was found that, with the present method, outstanding compaction was achieved with only one compacting step through the roller compactor. It is therefore preferable to compact L-tryptophan only once, in terms of efficiency and economy of the process, and also to minimize the risk of loss of chemical purity of the product.

### Pharmaceutical compositions

The authors of the present invention surprisingly discovered that by using the high-density L-tryptophan of the present invention as active ingredient, instead of common, low-density L-tryptophan, it was possible to prepare pharmaceutical compositions considerably reducing the amount of excipients needed for the formulation. Reductions of more than 20%, or more than 30%, or more than 40%, or even more than 50% in the amount of excipients used for a given dosage of L-tryptophan were achieved.

Example 2 shows the preparation of soft gelatin capsules containing a dose of 300 mg of L-tryptophan as active ingredient using high-density L-tryptophan (bulk density of 0.44 g/ml) as active ingredient, while Reference Example shows the preparation of an analogous formulation using low-density tryptophan (bulk density of 0.18 g/ml). The purpose when preparing both formulations was to use the minimum necessary amount of excipients to obtain a suitable formulation. It was surprisingly found that, when using the low-density form of the active ingredient, the total weight of the dosage form amounted to 1.5 g, while when high-density L-tryptophan according to the present invention was used, the weight of the final dosage form was 0.87 g. That means a reduction in the amount of excipients of 54%. Furthermore, it was found that both forms had equivalent properties in terms of uniformity and solubility, and both disintegrated in less than 30 minutes.

The possibility of reducing so drastically the amount of excipients needed in the formulation of the dosage forms entails both economic and therapeutic advantages. Thus, on the one hand, less excipients are required and also the production times are shortened. On the other hand, the reduction of the volume facilitates the ingestion of the dosage form by the patient and enables a better compliance of the therapeutic regime. Figure VI shows the soft gelatin capsules obtained in Reference Example and in Example 2, both containing 300 mg of L-tryptophan, but using low- and high-density tryptophan, respectively, wherein the outstanding volume reduction can be clearly observed.

Therefore, another aspect of the invention is the use of compacted L-tryptophan for the preparation of a pharmaceutical composition.

Another further aspect of the invention is a pharmaceutical composition comprising compacted L-tryptophan and at least one pharmaceutically acceptable excipient and/or vehicle.

Another aspect of the invention is a process for preparing a pharmaceutical composition comprising L-tryptophan and at least one pharmaceutically acceptable excipient comprising a step of previously compacting L-tryptophan by roller compaction.

It is understood that L-tryptophan is the active ingredient of the pharmaceutical composition. Optionally, the pharmaceutical composition can contain one or more additional active ingredients to reinforce the desired therapeutic activity of the composition.

The pharmaceutical composition comprising compacted high-density L-tryptophan as active ingredient, according to the present invention can be any conventional formulation which can be prepared using methods that are well known to the person skilled in pharmaceutical formulation, as those disclosed in handbooks of pharmaceutical technology, for example, in the book J.P Remington and A. R. Genaro, Remington The Science and Practice of Pharmacy, 20th edition, Lippincott, Williams & Wilkins, Philadelphia, 2000 [ISBN: 0-683-306472] or in the book M.E. Aulton and K.M.G. Taylor, Aulton's Pharmaceutics, the design and manufacture of medicines, 4th edition, Churchill Livingstone Elsevier, 2013 [ISBN: 978-0-7020-4290-4], or in the book A.K. Dash, S. Singh and J. Tolman, Pharmaceutics. Basic principles and application to pharmacy practice, Academic Press, Elsevier, 2014 [ISBN: 978-0-12-386890-9].

The excipients suitable to be used in the pharmaceutical compositions of the present invention are also well known to those skilled in pharmaceutical technology and are described, for example, in the book R.C. Rowe, P.J. Sheskey and P.J. Weller, Handbook of Pharmaceutical Excipients, Sixth Edition, Pharmaceutical Press, 2009.

The pharmaceutical compositions suitable to be prepared according to the present invention are all solid pharmaceutical compositions for oral use. Typically, the pharmaceutical composition is selected from tablets, soft gelatin capsules and hard gelatin capsules.

Tablets comprising high-density L-tryptophan can be formulated using standard procedures, well known in the art. Preferably, tablets are prepared by direct compression, i.e., high-density L-tryptophan is mixed with suitable excipients and compressed without any previous granulation process. Alternatively, high-density L-tryptophan can be previously granulated with additional excipients, either by wet granulation, fluid-bed granulation or dry granulation, and then optionally mixed with extra-granular excipients and compressed into tablets. Excipients typically used in the formulation of tablets are diluents, binding agents, glidants, lubricants, disintegrating agents, and mixtures thereof. Other additional excipients include sweetening agents, flavouring agents and colouring agents, for example.

Suitable diluents include calcium carbonate, calcium phosphate, calcium sulfate, cellulose acetate, dextrates, dextrins, dextrose, ethylcellulose, fructose, glyceryl palmitostearate, isomalt, kaolin, lactitol, lactose, magnesium carbonate, magnesium oxide, maltodextrins, maltose, mannitol, microcrystalline or powdered cellulose, pregelatinized starch, starch, sodium carbonate, sodium chloride, sorbitol and sucrose, among others, and mixtures thereof.

Suitable binding agents include acacia, agar, cellulose acetate phthalate, copovidone, dextrates, dextrin, dextrose, ethylcellulose, gelatin, glyceryl behenate, guar gum, hydroxyethyl cellulose, hydroxyethylmethyl cellulose, hydroxypropyl cellulose, hydroxypropylmethyl cellulose methylcellulose, maltodextrin, microcrystalline cellulose, povidone, pregelatinized starch, sodium carboxymethylcellulose, starch, stearic acid and sucrose, among others, and mixtures thereof.

Suitable glidants include powdered cellulose, colloidal silicon dioxide, magnesium oxide, magnesium silicate, magnesium trisilicate, silicon dioxide, and talc, among others, and mixtures thereof.

Suitable lubricants include calcium stearate, glyceryl behenate, glyceryl palmitostearate, hydrogenated castor oil, magnesium stearate, myristic acid, palmitic acid, polyethylene glycol, sodium benzoate, sodium lauryl sulfate, sodium stearyl fumarate, stearic acid, and talc, among others, and mixtures thereof.

Suitable disintegrants include alginic acid, crospovidone, sodium croscarmellose, sodium starch glycolate, starch, and low-substituted hydroxypropyl cellulose, among others, and mixtures thereof.

Furthermore, tablets may be formulated either as conventional compressed tablets, or in other forms, for example, buccal, sublingual, chewable, sustained-release or orally-disintegrating tablets, by selecting suitable excipients and manufacturing processes, as are well known in the art.

Capsules, as is well known in the pharmaceutical field, are solid dosage forms in which the drug substance is enclosed in either a hard or soft, soluble container or shell. The raw materials used in the manufacture of the shell are similar for both hard and soft gelatin capsules, while the proportions vary. The major component of a capsule shell is gelatin; other components include water, plasticizers, such as glycerine, sorbitol or propylene glycol, colorants, opacifiers, such as titanium dioxide, and preservatives. Hypromellose can alternatively be used as capsule shell material for hard capsules.

Hard capsules consist of two sections, one slipping over the other and thus enclosing the drug formulation, which is generally in solid form, as powder or granulate, or occasionally in liquid or semi-liquid form, while in soft gelatin capsules, also referred to as softgels, a continuous, one-piece, hermetically sealed gelatin shell surrounds the drug formulation, which is generally in liquid, suspension or semisolid form.

The powders or granules included within hard capsules comprise the active ingredient and typically at least one pharmaceutically acceptable excipient, generally selected from diluents, lubricants and glidants, and mixtures thereof, which are well-known in the art, for example, those disclosed above for the formulation of tablets.

The term "powders" typically refers to intimate mixtures of finely divided active ingredient, optionally with one or more excipients. The active ingredient and the excipient(s) are mixed to obtain an homogeneous mixture, for example using trituration, spatulation, sifting or tumbling procedures, which are well-known in the art.

The term "granules" typically refers to larger particles, either containing the active ingredient alone, or, more frequently, aggregations of active ingredient particles with additional excipients, typically prepared by dry granulation, wet granulation or fluid-bed granulation procedures, which are also well known in the art.

The fill compositions for soft gelatin capsules generally comprise the active ingredient, i.e., L-tryptophan, dissolved or dispersed in a liquid vehicle, generally, L-tryptophan is dispersed in the vehicle. The vehicle is typically an oily liquid, for example, a vegetable oil, such as peanut oil, castor oil, olive oil, rapeseed oil, corn oil, sesame oil, sunflower oil or soybean oil, among others, or medium chain triglycerides (MCT), or mixtures thereof. Other liquid, non-oily, vehicles can also be used, for example, polyethylene glycols, isopropyl alcohol, propylene glycol, glycerol, polyglycerols, triacetin, glyceryl esters, sorbitan esters, sugar esters and polyglyceryl esters, among others, or mixtures thereof. The composition generally additionally comprises other excipients, acting as suspending agents and/or viscosity modifying agents, for example, hydrogenated vegetable oils, waxes, or ethylcellulose for oily vehicles and solid glycol esters for non-oily vehicles. Surfactants such as lecithin or polysorbate 80 may also be added to improve the dispersion of the suspended drug particles. Alternatively, the fill composition may be in the form of self-emulsifying lipophilic systems or microemulsion pre-concentrates, which typically comprise a lipophilic solvent, such as a vegetable oil or a medium chain triglyceride, and a surfactant, and optionally a co-solvent, such as ethanol or propyleneglycol.

High-density L-tryptophan may also be formulated as powders or granules for oral administration. They may be directly administered to the oral cavity or either they can be previously dissolved or dispersed in water or in other liquids before being ingested. They may also be effervescent powders or granules.

In one embodiment, the pharmaceutical composition comprising high-density L-tryptophan and at least one pharmaceutically acceptable excipient is selected from the group consisting of tablets, hard capsules, soft capsules, powders and granules, and preferably is selected form tablets, hard capsules and soft capsules.

In a particularly preferred embodiment of the invention, the composition is in the form a soft gelatin capsule. Preferably, the fill composition comprises an oily vehicle selected from the group consisting of castor oil, olive oil, rapeseed oil, sesame oil, sunflower oil, soybean oil and mixtures thereof and optionally other auxiliary excipients selected from lecithin, polysorbate 80, wax, hydrogenated vegetable oils and mixtures thereof.

In one embodiment, each dosage form unit, either in the form of tablet, hard capsule or soft capsule, comprises a dose of L-tryptophan comprised in the range from about 100 to about 1000 mg, preferably from about 200 to about 800 mg, and more preferably from about 300 to about 700 mg, for example the dose may be selected from the following: about 100, 150, 200, 250, 300, 350, 400, 450, 500, 550, 600, 650, 700, 750, 800, 850, 900, 950 and 1000 mg. Preferred doses are about 300, 350, 400, 450, 500, 550, 600, 650 or 700 mg of L-tryptophan in each dosage form unit.

### Use

The pharmaceutical dosage forms prepared with compacted L-tryptophan have the advantage for the patient of being smaller than those prepared with conventional, low-density L-tryptophan. This fact contributes to a better patient compliance and, consequently, to improved therapeutic efficacy of the pharmacological treatment.

Therefore, another aspect of the present invention relates to a pharmaceutical composition comprising compacted L-tryptophan for use in therapy.

Any references in this description to methods of treatment refer to compounds, pharmaceutical compositions and medicaments of the present invention for use in a method for treatment of the human (or animal) body by therapy (or for diagnosis).

Another aspect of the invention is a method of treating a disease or condition susceptible to treatment with L-tryptophan in a subject, the method comprising administering a pharmaceutical composition comprising compacted L-tryptophan to the subject.

In particular, L-tryptophan may be used for the treatment of different diseases or conditions, including pain, insomnia, premenstrual syndrome, depression, fibromyalgia and obesity.

The recommended daily dose of L-tryptophan widely varies, depending on the particular disease or condition to be treated, as is well-known to the skilled in the art. For example, the recommended daily dosage of L-tryptophan may range from about 300 mg to about 10 g, preferably from about 500 mg to about 5 g.

The terms "treatment" or "treating" as used herein have their conventional meaning and typically refer to the administration of the pharmaceutical composition with the purpose of attenuating, alleviating, minimising, or suppressing the symptoms of the treated disease or condition.

The particular and preferred embodiments of the pharmaceutical composition regarding its use in therapy are the same as described above in the previous section.

### Examples

### Example 1.- Preparation of high-density L-tryptophan

For the preparation of high-density L-tryptophan, a L-tryptophan prepared by fermentation with a strain of *Corynebacterium glutamicum* (*C. Glutamicum* FC22) and employing cassava starch in the fermentation medium was used. It was a creamy granular powder and had a bulk density of 0.18 g/ml and particle size of less than 1 mm.

The purity of L-tryptophan before and after the compaction process was assessed by means of HPLC. The HPLC specifications used are as follows:

| | | |
|---|---|---|
| Equipment: | | 1220 Infinity LC (Agilent Technologies) |
| Column: | | ACE3 C18 PFP (150 x 4.6 mm) |
| Flow rate: | | 1 ml/min |
| Injection volume: | | 5 µl |
| Detector: | | 222 nm |
| Run time: | | 15 minutes |
| Solvent: | A: | water with 0.05% (v/v) formic acid |
| | B: | methanol |

Gradient:

| time [min] | solvent A [%] | solvent B [min] |
|---|---|---|
| 0.0 | 55 | 45 |
| 15 | 55 | 45 |

Both the original low-density L-tryptophan and the high-density L-tryptophan obtained after the compaction process fulfilled the requirements of the European Pharmacopoeia (Ph. Eur.), and had chemical purity greater than 98.5%, determined by HPLC.

For the densification process, a roller compactor equipment was used (Bonals BC-150/75V), provided with vacuum system for feeding the raw material into the screw feeding system. The roller compactor had one roll with smooth surface and one roll with knurled surface. The dimensions of both rolls were 10.5 cm diameter and 7.4 cm width.

The compactor was connected with a rotor granulator for milling the outcoming compacted plaque and the obtained granulate was passed through a sieve of 1 mm mesh. The granulation speed (GS) was fixed to 7.33 rad/s (70 r.p.m.).

The roll speed (RS) in the roller compactor was fixed to 0.73 rad/s (7 r.p.m.). Different compacting assays were performed by varying the compaction parameters, namely: Screw feeding speed (FS), roll force (RF) and roll gap (RG). The results are shown below, arranged in assays A to E.

### Assay A:

Several samples of high-density L-tryptophan were prepared, by fixing the roll gap to 0.5 mm and the roll force to 137209.31-147009.97 N (14000-15000 Kgf). Then, several values of feeding speed were assayed, for evaluating its influence on the bulk density of the compacted substance. The results are shown below in Table I.

**TABLE I**

| Sample | FS (r.p.m.) | Bulk density (g/ml) | Bulk density increase (%) |
|---|---|---|---|
| TEST-04 | 11 | 0.30 | 67 |
| TEST-05 | 20 | 0.33 | 83 |
| TEST-06 | 30 | 0.35 | 94 |
| TEST-07 | 40 | 0.37 | 106 |
| TEST-08 | 50 | 0.39 | 117 |
| TEST-09 | 60 | 0.42 | 133 |

It can be observed that higher densities were obtained with higher feed speed (FS) values.

The results of Table I are represented graphically in Figure I, where the x-axis represents the FS values (in r.p.m.) and the y-axis represents the bulk density values (in g/ml).

### Assay B

Several samples of high-density L-tryptophan were prepared, by fixing the roll gap to 0.5 mm and the roll force to 58803.99-68604.65 N (6000-7000 Kgf). Then, several values of the feeding speed were assayed, for evaluating its influence on the bulk density of the compacted substance. The results are shown below in Table II.

**TABLE II**

| Sample | FS (r.p.m.) | Bulk density (g/ml) | Bulk density increase (%) |
|---|---|---|---|
| TEST-18 | 11 | 0.22 | 22 |
| TEST-17 | 20 | 0.24 | 33 |
| TEST-16 | 30 | 0.26 | 44 |
| TEST-15 | 40 | 0.28 | 56 |
| TEST-14 | 50 | 0.33 | 83 |
| TEST-13 | 60 | 0.37 | 106 |

Again, as in Assay A, it can be observed that higher densities were obtained with higher feed speed (FS) values. The increase in densities is lower than in Assay A, so it follows that with higher roll force higher bulk density of the final product is obtained.

The results of Table II are represented graphically in Figure II, where the x-axis represents the FS values (in r.p.m.) and the y-axis represents the bulk density values (in g/ml).

### Assay C

In the following tests, the speed of the feed screw remained constant at 1.15 rad/s (11 r.p.m.) and the roll gap was also fixed at 0.5 mm in all the tests. The roll force was varied from 58803.99-68604.65 N (6000-7000 Kgf) to 137209.31-147009.97 N (14000-15000 Kgf), to examine its influence on the density of the final product. The results are shown in Table III:

**TABLE III**

| Sample | RF (Kgf) | Bulk density (g/ml) | Bulk density increase (%) |
|---|---|---|---|
| TEST-18 | 6000-7000 | 0.22 | 22 |
| TEST-01 | 8000-9000 | 0.25 | 39 |
| TEST-02 | 10000-11000 | 0.29 | 61 |
| TEST-03 | 12000-13000 | 0.30 | 67 |
| TEST-04 | 14000-15000 | 0.30 | 67 |

It is apparent from the results than higher roll forces are preferable in order to obtain higher bulk density increases. The results of of Table III are represented graphically in Figure III, where the x-axis represents the RF values (in Kgf) and the y-axis represents the bulk density values (in g/ml).

### Assay D

In the following tests, the speed of the feed screw remained constant at 6.28 rad/s (60 r.p.m.) and the roll gap was also fixed at 0.5 mm in all the tests. The roll force was varied from 58803.99-68604.65 N (6000-7000 Kgf) to 137209.31-147009.97 N (14000-15000 Kgf), to assess its influence on the density of the final product. The results are shown in Table IV:

**TABLE IV**

| Sample | RF (Kgf) | Bulk density (g/ml) | Bulk density increase (%) |
|---|---|---|---|
| TEST-13 | 6000-7000 | 0.37 | 106 |
| TEST-12 | 8000-9000 | 0.39 | 117 |
| TEST-11 | 10000-11000 | 0.40 | 122 |
| TEST-10 | 12000-13000 | 0.41 | 128 |
| TEST-09 | 14000-15000 | 0.42 | 133 |

Again, as in Assay C, it can be observed that higher densities were obtained with higher roll forces (RF) values. The increase in densities is higher than in Assay C, so it follows that higher values of food supply (FS) are preferable for obtaining higher density increases.

The results of Table IV are represented graphically in Figure IV, where the x-axis represents the RF values (in Kgf) and the y-axis represents the bulk density values (in g/ml).

### Assay E

In the following tests, the feed supply remained constant at 6.28 rad/s (60 r.p.m.) and the roll force also remained constant at 137209.31-147009.97 N (14000-15000 Kgf), whereas the roll gap (RG) was varied from 0,3 to 0,9 mm, to examine its influence on the density of the final product. The results are shown in Table V:

**TABLE V**

| Sample | RG | Bulk density (g/ml) | Bulk density increase (%) |
|---|---|---|---|
| TEST-19 | 0.3 | 0.27 | 50 |
| TEST-20 | 0.4 | 0.33 | 83 |
| TEST-21 | 0.5 | 0.44 | 144 |
| TEST-22 | 0.6 | 0.41 | 128 |
| TEST-23 | 0.7 | 0.36 | 100 |
| TEST-24 | 0.8 | 0.34 | 89 |
| TEST-25 | 0.9 | 0.31 | 72 |

It can be observed that the maximum density was obtained when the roll gap was 0.5 mm.

The results of Table V are represented graphically in Figure V, where the x-axis represents the RG values (in mm) and the y-axis represents the bulk density values (in g/ml).

### Example 2.- Preparation of soft gelatin capsules using high-density L-tryptophan

Soft gelatin capsules containing high-density L-tryptophan were prepared. The high-density L-tryptophan used for preparing this formulation was that obtained in Example 1 (Test 21), which had a bulk density of 0.44 g/ml and a particle size of less than 1 mm.

The fill composition to be enclosed within the capsules comprised refined sunflower oil, sunflower lecithin, hydrogenated palm oil (Softisan^{®} 154), and yellow wax. The shell contained gelatin, glycerin and colorants.

Each capsule contained 300 mg of active ingredient, i.e., high-density L-tryptophan. The formulation was designed with the purpose of preparing capsules using the minimum possible amount of excipients, but without undermining the desirable quality of the formulation, namely, good uniformity, stability, appearance and solubility, and also good dissolution/dispersion of the active ingredient in the oily matrix.

The quantitative formulation of each finished capsule is shown in Table VI below:

**TABLE VI**

| **Ingredient** | **mg/capsule** |
|---|---|
| High-density L-tryptophan | 300 |
| Refined sunflower oil | 280 |
| Sunflower lecithin | 35 |
| Softisan^{®} 154 | 10 |
| Yellow wax | 5 |
| Total fill content | 630 |
| Surrounding shell | 245 |
| **Total capsule** | **875** |

The composition of the surrounding shell was approximately as follows: gelatin (64.9%), glycerin (28.4%), water (6.0%), titanium dioxide E-171 (0.6%) and brilliant blue E-133 (0.1%).

The preparation of the soft gelatin capsules was performed employing conventional methods, which are well-known in the art. Thus, the fill composition was prepared by mixing all the excipients (oil, lecithin, Softisan and wax), in a mixer/homogenizer apparatus, until a homogeneous mixture was obtained. The high-density L-tryptophan was subsequently added, and thoroughly mixed until a homogeneous suspension as obtained.

Gelatin, water and glycerin were mixed and heated to 80° C, the colorants were then added, and the gel mass was supplied to the encapsulation machine, so two continuous gel ribbons were carried through counter-rotating rollers to the rotary die encapsulation tool and a wedge-shaped injection system between the rolls with a precision nozzle allowed injecting metered volumes of the liquid fill composition inside the capsules. The finished capsules were cooled and dried.

Blue coloured, 12 oval, soft gelatin capsules were obtained. The amount of fill material in each capsule was 630 mg, with a variation of ± 5% (598.5-281.4 mg). The amount of shell material in each capsule was 244.669 mg, with a variation of ± 15% (208.0 - 281.4 mg). The total capsule weight was 874.669 mg, with a variation of ± 7.5% (90%) or ± 15% (100%).

The disintegration time was 30 minutes or less, according to the disintegration test described in the European Pharmacopoeia 9th Edition, section 2.9.1, Disintegration of tablets and capsules, 299-366.

### Reference Example.- Preparation of soft gelatin capsules using low-density L-tryptophan

A comparative formulation comprising 300 mg of L-tryptophan was prepared, analogously as disclosed in Example 2, but using low-density L-tryptophan, having a bulk density of 0.18 g/ml, instead of high-density L-tryptophan.

Qualitatively, the excipients used were the same as those disclosed above for Example 2. Analogously as in Example 2, the purpose of the formulation was to use the minimum possible amount of excipients necessary to obtain soft gelatin capsules with suitable formulation properties.

The quantitative formulation of each finished capsule is shown in Table VII below:

**TABLE VII**

| **Ingredient** | **mg/capsule** |
|---|---|
| Low-density L-tryptophan | 300 |
| Refined sunflower oil | 735 |
| Sunflower lecithin | 50 |
| Softisan^{®} 154 | 10 |
| Yellow wax | 5 |
| Total fill content | 1100 |
| Surrounding shell | 438 |
| **Total capsule** | **1538** |

The composition of the shell was analogous as that disclosed above in Example 2. The capsules were also prepared following an analogous method as disclosed in Example 2.

Blue coloured, 20 oblong, soft gelatin capsules were obtained. The amount of fill material in each capsule was 1100 mg, with a variation of ± 5% (1045 -1155 mg). The amount of shell material in each capsule was 437.790 mg, with a variation of ± 15% (372.1 - 503.5 mg). The total capsule weight was 1537.790 mg, with a variation of ± 7.5% (90%) or ± 15% (100%).

The disintegration time was 30 minutes or less, according to the disintegration test described in the European Pharmacopoeia 9th Edition, section 2.9.1, Disintegration of tablets and capsules, 299-366.

The weight and volume difference between the capsules obtained using common low-density L-tryptophan versus those capsules prepared with high-density L-tryptophan according to the present invention is outstanding.

Thus, by using high-density L-tryptophan instead of low-density L-tryptophan, the total reduction of excipients was of about 54%. Namely, the reduction of excipients in the fill composition was of about 59%, and the amount of glycerin and gelatin in the shell was reduced in about 44%.

Consequently, the size of the capsule was also remarkably reduced, as is shown in Figure VI wherein the capsules prepared in Example 2 and in Reference Example are shown, both containing the same amount of active ingredient.

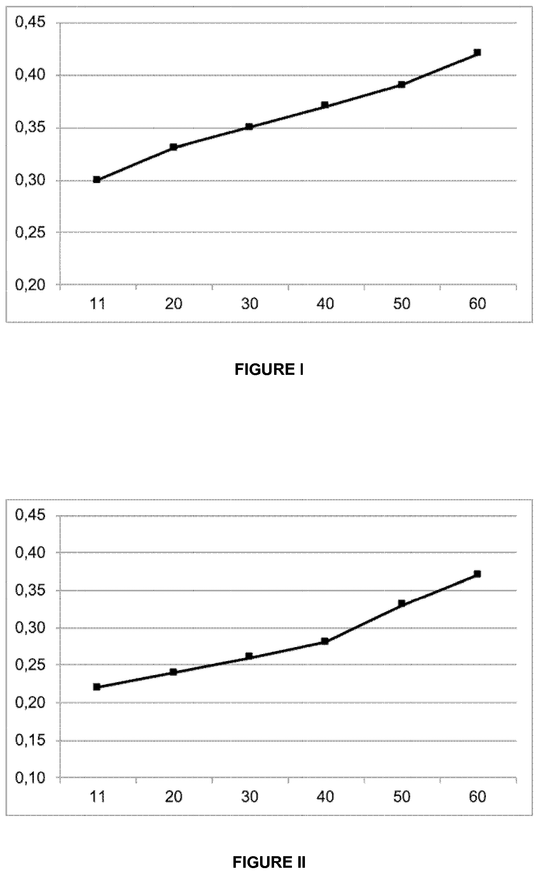

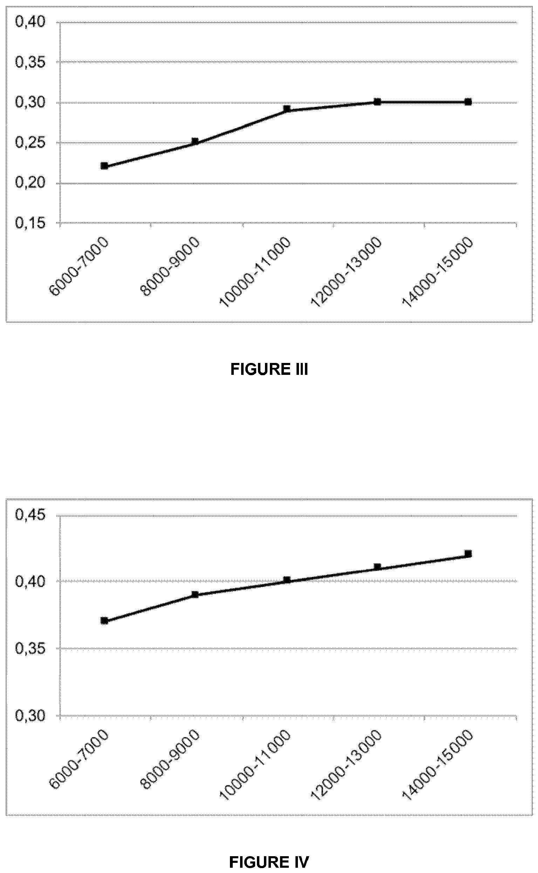

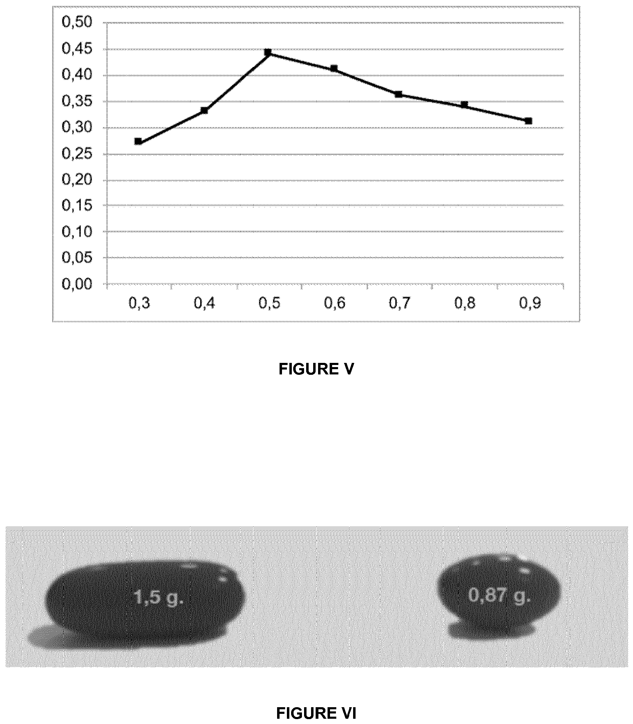

## Claims

1. A process for preparing substantially pure compacted L-tryptophan comprising roller compaction of L-tryptophan, wherein substantially pure means a purity of at least 98%, and preferably of at least 98.5%, and wherein L-tryptophan is compacted alone, without adding any auxiliary excipient.

2. Process according to claim 1, **characterized in that** a roller compactor is used provided with a screw feeder and the feed speed is adjusted to a value comprised between 3.14 rad/s and 7.33 rad/s (30 and 70 r.p.m.).

3. Process according to claim 1 or 2, **characterized in that** a vacuum system is employed for feeding L-tryptophan raw material into the screw feeder.

4. Process according to any one of claims 1 to 3, **characterized in that** the roll speed is adjusted to a value comprised between 0.52 rad/s and 1.57 rad/s (5 and 15 r.p.m.).

5. Process according to any one of claims 1 to 4, **characterized in that** the roll gap is adjusted to a value comprised between 0.3 and 0.9 mm.

6. Process according to any one of claims 1 to 5, **characterized in that** the roll force is adjusted to a value comprised between 49003.32 N and 156810.64 N (5000 and 16000 Kgf).

7. Process according to any one of claims 1 to 6, **characterized in that** one of the rolls has a smooth surface and the other has a non-smooth surface.

8. Compacted L-tryptophan which is obtainable by a process according to any one of claims 1 to 7 and having a bulk density equal to or higher than 0.32 g/ml

9. Compacted L-tryptophan according to claim 8, **characterized in that** it has a bulk density higher than 0.40 g/ml.

10. Compacted L-tryptophan according to any one of claims 8 to 9, **characterized in that** it has a particle size of less than 1 mm.

11. Use of compacted L-tryptophan according to any one of claims 8 to 10 for the preparation of a pharmaceutical composition.

12. Pharmaceutical composition comprising compacted L-tryptophan according to any one of claims 8 to 10 and at least one pharmaceutically acceptable excipient and/or vehicle.

13. Pharmaceutical composition according to claim 12 for use in therapy.

## Patentansprüche

1. Verfahren zur Herstellung von im Wesentlichen reinem verdichtetem L-Tryptophan umfassend Walzenverdichtung von L-Tryptophan, wobei im Wesentlichen rein eine Reinheit von mindestens 98%, und vorzugsweise von mindestens 98,5%, bedeutet, und wobei das L-Tryptophan allein verdichtet wird, ohne einem zusätzlichen Hilfsstoff hinzuzufügen.

2. Verfahren nach Anspruch 1, **dadurch gekennzeichnet, dass** ein Walzenverdichter, welcher mit einem Schneckenzuführer versehen ist, verwendet wird und dass die Zuführgeschwindigkeit auf einen Wert, welcher zwischen 3,14 rad/s und 7,33 rad/s (30 und 70 U/min) liegt, eingestellt wird.

3. Verfahren nach Anspruch 1 oder 2, **dadurch gekennzeichnet, dass** ein Vakuumsystem zum Zuführen von L-Tryptophan-Ausgangsmaterial in den Schneckenzuführer verwendet wird.

4. Verfahren nach einem der Ansprüche 1 bis 3, **dadurch gekennzeichnet, dass** die Walzengeschwindigkeit auf einen Wert, welcher zwischen 0,52 rad/s und 1,57 rad/s (5 und 15 U/min) liegt, eingestellt wird.

5. Verfahren nach einem der Ansprüche 1 bis 4, **dadurch gekennzeichnet, dass** der Walzenspalt auf einen Wert, welcher zwischen 0,3 und 0,9 mm liegt, eingestellt wird.

6. Verfahren nach einem der Ansprüche 1 bis 5, **dadurch gekennzeichnet, dass** die Walzenkraft auf einen Wert, welcher zwischen 49003,32 N und 156810,64 N (5000 und 16000 kgf) liegt, eingestellt wird.

7. Verfahren nach einem der Ansprüche 1 bis 6, **dadurch gekennzeichnet, dass** eine der Walzen eine glatte Oberfläche aufweist und die andere eine nicht-glatte Oberfläche aufweist.

8. Verdichtetes L-Tryptophan, welches mittels eines Verfahrens nach einem der Ansprüche 1 bis 7 erhältlich ist und eine Schüttdichte gleich oder größer als 0,32 g/ml aufweist.

9. Verdichtetes L-Tryptophan nach Anspruch 8, **dadurch gekennzeichnet, dass** es eine Schüttdichte höher als 0,40 g/ml aufweist.

10. Verdichtetes L-Tryptophan nach einem der Ansprüche 8 bis 9, **dadurch gekennzeichnet, dass** es eine Partikelgröße kleiner als 1 mm aufweist.

11. Verwendung von verdichtetem L-Tryptophan nach einem der Ansprüche 8 bis 10 zur Herstellung einer pharmazeutischen Zusammensetzung.

12. Pharmazeutische Zusammensetzung umfassend verdichtetes L-Tryptophan nach einem der Ansprüche 8 bis 10 und mindestens einen pharmazeutisch akzeptablen Hilfsstoff und/oder Träger.

13. Pharmazeutische Zusammensetzung nach Anspruch 12 für deren Verwendung in Therapie.

## Revendications

1. Procédé pour la préparation de L-tryptophane compacté substantiellement pur comprenant le compactage à rouleaux de L-tryptophane, dans lequel substantiellement pur signifie une pureté d'au moins 98 %, et préférablement d'au moins 98,5 %, et dans lequel le L-tryptophane est compacté seul, sans ajout d'aucun excipient auxiliaire.

2. Procédé selon la revendication 1, **caractérisé en ce qu'**un compacteur à rouleaux est utilisé muni d'un alimenteur à vis et que la vitesse d'alimentation est ajustée à une valeur comprise entre 3,14 rad/s et 7,33 rad/s (30 et 70 tr/min).

3. Procédé selon revendication 1 ou 2, **caractérisé en ce qu'**un système de vide est employé pour alimenter en matière première de L-tryptophane dans l'alimentateur à vis.

4. Procédé selon l'une quelconque des revendications 1 à 3, **caractérisé en ce que** la vitesse des rouleaux est ajustée à une valeur comprise entre 0,52 rad/s et 1,57 rad/s (5 et 15 tr/min).

5. Procédé selon l'une quelconque des revendications 1 à 4, **caractérisé en ce que** l'écartement des rouleaux est ajusté à une valeur comprise entre 0,3 et 0,9 mm.

6. Procédé selon l'une quelconque des revendications 1 à 5, **caractérisé en ce que** la force des rouleaux est ajustée à une valeur comprise entre 49003,32 N et 156810,64 N (5000 et 16000 Kgf).

7. Procédé selon l'une quelconque des revendications 1 à 6, **caractérisé en ce qu'**un des rouleaux a une surface lisse et l'autre une surface non lisse.

8. L-tryptophane compacté qui est obtenable par un procédé selon l'une quelconque des revendications 1 à 7 et ayant une densité apparente égale ou supérieure à 0,32 g/ml.

9. L-tryptophane compacté selon la revendication 8, **caractérisé en ce qu'**il a une densité apparente supérieure à 0,40 g/ml.

10. L-tryptophane compacté selon l'une quelconque des revendications 8 à 9, **caractérisé en ce que** qu'il a une taille de particules de moins de 1 mm.

11. Utilisation de L-tryptophane compacté selon l'une quelconque des revendications 8 à 10 pour la préparation d'une composition pharmaceutique.

12. Composition pharmaceutique comprenant L-tryptophane compacté selon l'une quelconque des revendications 8 à 10 et au moins un excipient et/ou un véhicule pharmaceutiquement acceptable.

13. Composition pharmaceutique selon la revendication 12 pour utilisation en thérapie.
